# EUROPEAN PATENT APPLICATION

(11) **EP 4 375 377 A1**
(43) Date of publication of application: **29.05.2024**
(21) Application number: 22845757.8
(22) Date of filing: 29.06.2022
(51) Int. Cl.: C12N 15/55, C12Q 1/34, C12N 9/80

(54) **SUBSTANCE DETECTION METHOD USING AMINOACYLASE**

(30) Priority: 19.07.2021 JP 2021118420
(71) Applicant: Josho Gakuen Educational Foundation, Osaka 535-8585 (JP); Nipro Corporation, Settsu-shi, Osaka 566-8510 (JP)
(72) Inventor: NISHIYA, Yoshiaki, Neyagawa-shi, Osaka 572-8508 (JP); TATSUMI, Kenta, Osaka-shi, Osaka 531-8510 (JP); YAGURA, Kazuki, Osaka-shi, Osaka 531-8510 (JP)
(74) Representative: Dantz, Jan Henning
(86) International application number: PCT/JP2022/025936
(87) International publication number: WO 2023/002821

(57) **Abstract**

At least the presence of a starting material is detected by measuring glycine formed as a by-product along with a main product through a chemical reaction involving hippuric acid and/or methylhippuric acid as the starting material and catalyzed by a hydrolase. The hydrolase is an aminoacylase having an amino acid sequence of SEQ ID NO: 1 or a protein having the amino acid sequence of SEQ ID NO: 1 in which one or more amino acids are deleted, replaced, or added and having activity for hydrolysis of the hippuric acid and the methylhippuric acid.

## Description

### Technical Field

The present invention relates to a material detection method using an aminoacylase and particularly relates to a material detection method for detecting hippuric acid or methylhippuric acid by detecting glycine formed as a by-product along with a main product through a chemical reaction involving hippuric acid or methylhippuric acid as a starting material and catalyzed by an aminoacylase.

### Background Art

Hippuric acid is known as a metabolite of toluene, and methylhippuric acid is known as a metabolite of xylene. Hippuric acid is used as an indicator of toluene exposure, and methylhippuric acid is used as an indicator of xylene exposure. Detection of hippuric acid or methylhippuric acid is useful for clinical tests of workers who handle these organic solvents.

The present inventors have proposed a material detection method using glycine oxidase, and this method is disclosed in Patent Literature 1. In this method, glycine formed as a by-product along with a main product through a chemical reaction of a starting material is oxidized not by an enzymatic cycling reaction but by a direct reaction using the catalytic action of glycine oxidase, and hydrogen peroxide resulting from the oxidation is measured.

Patent Literature 1 mentions hippuric acid and methylhippuric acid as typical examples of the starting material in the chemical reaction of the disclosed material detection method. In the chemical reaction, hippuric acid or methylhippuric acid is treated with an aminoacylase or an analogous enzyme and thus hydrolyzed to form benzoic acid or methylbenzoic acid as a main product and glycine as a by-product.

As previously stated, the glycine formed as a by-product is enzymatically treated with glycine oxidase to form hydrogen peroxide. The hydrogen peroxide is measured, and thus the starting material (hippuric acid or methylhippuric acid) can be detected. The material detection method disclosed in Patent Literature 1 is not limited to detection of hippuric acid or methylhippuric acid but can be suitably used for detection of hippuric acid or methylhippuric acid.

### Citation List

### Patent Literature

PTL 1: Japanese Laid-Open Patent Application Publication No. 2019-187285

### Summary of Invention

### Technical Problem

The present inventors further investigated the use of the material detection method of Patent Literature 1 for detection of hippuric acid or methylhippuric acid. As a result, it has been found that the method leaves room for improvement in terms of reliable measurement of the total amount of hippuric acid and methylhippuric acid in a specimen.

Hitherto known enzymes that hydrolyze hippuric acid or methylhippuric acid include the aminoacylase (EC 3.5.1.14) as mentioned above, hippurate hydrolase (EC 3.5.1.32), and N-acyl-D-amino-acid deacylase (EC 3.5.1.81). An investigation by the present inventors has revealed that these hitherto known enzymes cannot well hydrolyze the ortho isomer of three isomers of methylhippuric acid.

Methylhippuric acid can be present in three isomeric forms, namely, ortho, meta, and para isomers. The known enzymes such as the above aminoacylase can well hydrolyze hippuric acid, 3-methylhippuric acid which is the meta isomer, and 4-methylhippuric acid which is the para isomer, but cannot well hydrolyze 2-methylhippuric acid which is the ortho isomer. If 2-methylhippuric acid cannot be well hydrolyzed, glycine derived from 2-methylhippuric acid cannot be successfully formed as a by-product, and this makes it difficult to appropriately determine the total amount of hippuric acid and methylhippuric acid in a specimen.

The present invention has been made to solve the problem as described above, and an object of the present invention is to provide a material detection method using an aminoacylase and able to determine the total amount of at least hippuric acid and methylhippuric acid.

### Solution to Problem

To solve the problem as described above, a material detection method according to the present disclosure includes detecting at least a presence of a starting material by measuring glycine formed as a by-product along with a main product through a chemical reaction involving the starting material and catalyzed by a hydrolase, wherein the starting material includes hippuric acid and/or methylhippuric acid, and the hydrolase is an aminoacylase having an amino acid sequence of SEQ ID NO: 1 or a protein having the amino acid sequence of SEQ ID NO: 1 in which one or more amino acids are deleted, replaced, or added and having activity for hydrolysis of the hippuric acid and the methylhippuric acid.

In the material detection method as defined above, an aminoacylase having the amino acid sequence of SEQ ID NO: 1 or a homologous sequence is used as an enzyme that hydrolyzes hippuric acid and methylhippuric acid, and for this reason 2-methylhippuric acid, which cannot be well hydrolyzed by conventional enzymes, can be hydrolyzed as well as hippuric acid. Thus, hippuric acid and all of the three isomers of methylhippuric acid can be well hydrolyzed. This makes it possible not only to determine the total amount of hippuric acid and methylhippuric acid but also to determine the amount of methylhippuric acid by subtracting the amount of hippuric acid from the determined total amount.

In the material detection method as defined above, the measuring of the glycine may be accomplished by measuring hydrogen peroxide formed from the glycine.

In the material detection method as defined above, the hydrogen peroxide may be formed by an enzymatic reaction catalyzed by glycine oxidase.

In the material detection method as defined above, the measuring of the hydrogen peroxide may be accomplished by reacting the hydrogen peroxide, aminoantipyrine, and a Trinder reagent under catalytic action of a peroxidase to form a quinone pigment and by subsequently measuring a degree of coloring attributed to the quinone pigment.

In the material detection method as defined above, the main product may include benzoic acid or methylbenzoic acid.

In the material detection method as defined above, a total amount of the hippuric acid and the methylhippuric acid contained in a sample that is an object of detection may be determined by the measuring of the hydrogen peroxide.

In the material detection method as defined above, an amount of the methylhippuric acid may be obtained by subtracting an amount of the hippuric acid from the determined total amount of the hippuric acid and the methylhippuric acid.

The present disclosure covers a material detection kit for use in the material detection method as defined above, the material detection kit including an enzyme that hydrolyzes the starting material, wherein the enzyme is an aminoacylase having an amino acid sequence of SEQ ID NO: 1 or a protein having the amino acid sequence of SEQ ID NO: 1 in which one or more amino acids are deleted, replaced, or added and having activity for hydrolysis of the hippuric acid and the methylhippuric acid.

The above and further objects, features and advantages of the present invention will be more apparent from the following detailed description of a preferred embodiment with reference to the accompanying drawings.

### Advantageous Effects of Invention

The present invention as defined above has the advantage of providing a material detection method using an aminoacylase and able to determine the total amount of at least hippuric acid and methylhippuric acid.

### Brief Description of Drawings

FIG. 1 is a schematic diagram illustrating the outline of a material detection method which is a typical example of the present disclosure and in which the starting material includes hippuric acid and methylhippuric acid.
FIG. 2 is a schematic diagram illustrating a typical example of a specific glycine measurement method used in the material detection method illustrated in FIG. 1.
FIG. 3 is a graph showing a comparison between measured concentrations of hippuric acid or methylhippuric acid in specimens and known concentrations of hippuric acid or methylhippuric acid in the specimens for an example where the material detection method according to the present disclosure was used.
FIG. 4 is a graph showing a comparison between measured concentrations of hippuric acid or methylhippuric acid in specimens and known concentrations of hippuric acid or methylhippuric acid in the specimens for a comparative example where a conventional aminoacylase was used.
FIG. 5 is a graph showing a comparison between measured concentrations of hippuric acid or methylhippuric acid in specimens and known concentrations of hippuric acid or methylhippuric acid in the specimens for a comparative example where hippurate hydrolase was used.

### Description of Embodiments

A material detection method according to the present disclosure is a method that includes detecting the presence of a starting material or the production of a main product by measuring glycine formed as a by-product along with the main product through a chemical reaction involving the starting material and catalyzed by a hydrolase. The starting material is hippuric acid or methylhippuric acid or includes both hippuric acid and methylhippuric acid. The hydrolase (aminoacylase) used to catalyze the chemical reaction of hippuric acid or methylhippuric acid is an amidohydrolase derived from *Staphylococcus aureus* COL or a mutant enzyme of the amidohydrolase. The amidohydrolase has activity for hydrolysis of hippuric acid and methylhippuric acid and, in particular, has activity for hydrolysis of all of the three isomers of methylhippuric acid.

### [Basic Features of Material Detection Method]

First, the basic features of the material detection method according to the present disclosure will be described. As mentioned above, the starting material to be detected by the material detection method according to the present disclosure includes hippuric acid and/or methylhippuric acid (that is, at least one of hippuric acid and methylhippuric acid), and the material detection method according to the present disclosure uses a catalytic reaction (enzymatic reaction) in which hippuric acid or methylhippuric acid is hydrolyzed with an aminoacylase. Hippuric acid is known as a metabolite of toluene, and methylhippuric acid is known as a metabolite of xylene.

Hippuric acid has the IUPAC name benzoylaminoacetic acid and is known also as N-benzoylglycine. Methylhippuric acid has a structure consisting of hippuric acid modified with a methyl group at the ortho, meta, or para position of the benzene ring. For example, methylhippuric acid with the methyl group at the ortho position has the IUPAC name (2-methylbenzoylamino)acetic acid and is known also as 7V-(o-toluoyl)glycine.

An aminoacylase (EC 3.5.1.14) catalyzes a reaction in which hippuric acid or methylhippuric acid (or each of hippuric acid and methylhippuric acid) is decomposed into benzoic acid or methylbenzoic acid and glycine. Known examples of enzymes that catalyze this reaction (enzymes analogous to the aminoacylase) include hippurate hydrolase (EC 3.5.1.32) and N-acyl-D-amino-acid deacylase (EC 3.5.1.81). In the present disclosure, as described later, the aminoacylase used is an amidohydrolase derived from *Staphylococcus aureus* COL or a mutant enzyme of the amidohydrolase.

As previously stated, hippuric acid is a metabolite of toluene, and methylhippuric acid is a metabolite of xylene. In the event that a worker handling toluene or xylene inhales this organic solvent, most of it is metabolized into benzoic acid or methylbenzoic acid in the worker's body, and then the benzoic acid or methylbenzoic acid undergoes glycine conjugation that produces hippuric acid or methylhippuric acid, which is excreted through urine. For this reason, hippuric acid or methylhippuric acid (which may be simply referred to as "hippuric acid or the like" hereinafter) is used as an indicator of exposure to toluene or xylene. Thus, in the present disclosure, examples of the sample subjected to the chemical reaction that forms glycine as a by-product include biological samples such as urine which contains (or may contain) hippuric acid or the like.

The sample subjected to the chemical reaction is not limited to urine and may be blood, saliva, spinal fluid, or interstitial fluid. When the sample is blood, it may be whole blood or a blood sample containing only some blood components. The same is true of other body fluid samples such as spinal fluid and interstitial fluid. An example of the origin of the sample is a worker, namely a human, who handles organic solvents. Thus, the material detection method according to the present disclosure can be suitably used for purposes such as a health checkup in which a human who handles organic solvents is tested for exposure to toluene or xylene. The origin of the sample is not limited to a human and may be any other animal (a mammal such as a dog, cat, pig, cow, rat, or mouse or a vertebrate other than mammals).

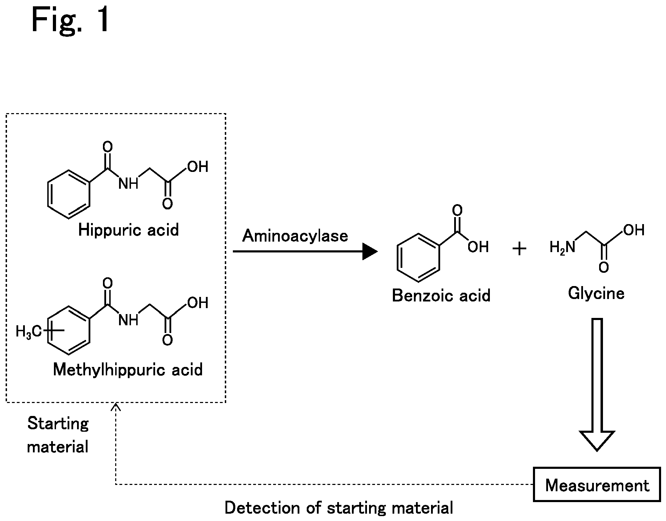

In the material detection method according to the present disclosure, as shown in FIG. 1, a certain amount of sample which contains (or may contain) hippuric acid or the like is collected, and an aminoacylase is added and mixed with the sample to hydrolyze hippuric acid or the like. As a result, benzoic acid or methylbenzoic acid (which may be hereinafter referred to as "benzoic acid or the like") and glycine are formed. The glycine is measured to detect the hippuric acid or the like which is the starting material. In the present disclosure, the method for measuring glycine is not limited to using a particular technique, and any hitherto known method can be suitably used. Typical examples of the method for measuring glycine include the below-described method in which hydrogen peroxide formed under the catalytic action of glycine oxidase is measured.

The enzyme used in the material detection method according to the present disclosure to hydrolyze the starting material is an enzyme capable of hydrolyzing hippuric acid or the like into benzoic acid or the like and glycine, i.e., an aminoacylase or the like. In the present disclosure, examples of the enzyme include: an aminoacylase having the amino acid sequence of SEQ ID NO: 1; and a protein having the amino acid sequence of SEQ ID NO: 1 in which one or more amino acids are deleted, replaced, or added and having activity for hydrolysis of hippuric acid and methylhippuric acid. The aminoacylase used is typically an amidohydrolase derived from *Staphylococcus aureus* (*S. zureus*) COL or a mutant enzyme of the amidohydrolase.

### [Features of Amidohydrolase]

Hereinafter, an amidohydrolase suitable for use as the hydrolase (aminoacylase) in the material detection method according to the present disclosure will be described in detail. Specific examples of the above-mentioned amidohydrolase derived from *S*. *zureus* COL include an amidohydrolase described in Tavarekere S. Girish, Vivek B, Melwin Colaco, Sandra Misquith, B. Gopal, "Structure of an amidohydrolase, SACOL0085, from methicillin-resistant Staphylococcus aureus COL" Acta Crystallographica Section F, 69, pp. 103-108, (2013) (Reference Literature 1). The contents of Reference Literature 1 are incorporated herein by reference.

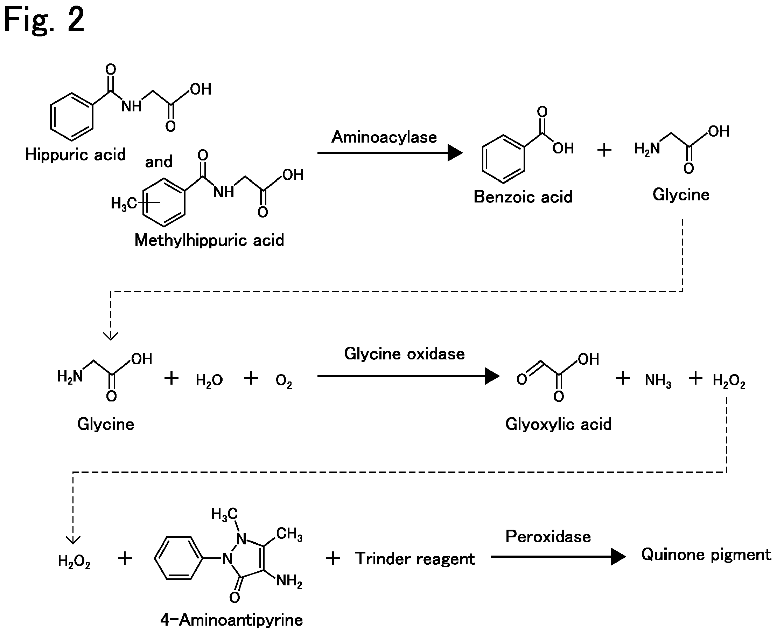

The amidohydrolase used in the present disclosure is an amino acid shown as "SACOL0085" in Figure 2 of Reference Literature 1 or a protein having the amino acid sequence of SEQ ID NO: 1. Alternatively, the amidohydrolase used in the present disclosure may be a protein having the amino acid sequence of SEQ ID NO: 1 in which one or more amino acids are deleted, replaced, or added and having activity for hydrolysis of hippuric acid and methylhippuric acid. The "one or more amino acids" may be, for example, 1 to 20 amino acids, 1 to 10 amino acids, 1 to 8 amino acids, 1 to 5 amino acids, or 1 to 3 amino acids.

Alternatively, the amidohydrolase used in the present disclosure may be a protein having an amino acid sequence that is at least 90% homologous to the amino acid sequence of SEQ ID NO: 1 and having activity for hydrolysis of hippuric acid and methylhippuric acid (this activity is referred to as "hydrolysis activity for hippuric acid and the like"). The sequence homology to the amino acid sequence of SEQ ID NO: 1 may be 95% or more, 97% or more, 98% or more, or 99% or more.

When the amidohydrolase used in the present disclosure is a protein having an amino acid sequence homologous to the amino acid sequence of SEQ ID NO: 1 and having hydrolysis activity for hippuric acid and the like, the "hydrolysis activity for hippuric acid and the like" refers to activity by which all of the three isomers of methylhippuric acid are hydrolyzed as well as hippuric acid. As experimentally demonstrated in Example described later, the amidohydrolase used in the present disclosure has hydrolysis activity not only for hippuric acid but also for all of 2-methylhippuric acid, 3-methylhippuric acid, and 4-methylhippuric acid. Thus, the total amount of hippuric acid and methylhippuric acid can be easily determined.

When the amidohydrolase used in the present disclosure is a protein having the amino acid sequence of SEQ ID NO: 1 or a homologous sequence (amino acid sequence of SEQ ID NO: 1 in which one or more amino acids are deleted, replaced, or added or amino acid sequence that is at least 90% homologous to the amino acid sequence of SEQ ID NO: 1), some of the amino acids may be altered to change the physical or chemical properties of the protein while maintaining the activity for hydrolysis of hippuric acid and methylhippuric acid.

Examples of the change of the physical or chemical properties of the protein include, but are not limited to, increase in thermal stability, change of substrate specificity, and change of optimum pH. Examples of the amino acid alteration include, but are not limited to, alteration not into L-amino acids but into D-amino acids, alteration into amino acids other than 20 to 22 amino acids which constitute proteins, and post-translational modifications to amino acids.

The method for producing the amidohydrolase used in the present disclosure is not limited to using a particular technique, and any hitherto known method can be suitably used. Typical examples include a method in which DNA having the amino acid sequence of SEQ ID NO: 1 or a homologous sequence is introduced into and overexpressed in host cells.

Examples of the DNA encoding the amidohydrolase used in the present disclosure include DNA having the base sequence of SEQ ID NO: 2. The base sequence of SEQ ID NO: 2 is available by searching the DBGET Search of KEGG (Kyoto Encyclopedia of Genes and Genomes, URL: https://www.genome.jp/kegg/) by the word "SACOL0085" (https://www.genome.jp/dbget-bin/www_bget?sac: SACOL0085). The amino acid sequence of SEQ ID NO: 1 is also available through the DBGET Search.

The DNA encoding the amidohydrolase used in the present disclosure may be any DNA that encodes the amino acid sequence of SEQ ID NO: 1 and is not limited to the DNA having the base sequence of SEQ ID NO: 2. For example, the DNA encoding the amidohydrolase used in the present disclosure may be DNA having a base sequence homologous to the base sequence of SEQ ID NO: 2 or DNA having another base sequence that encodes the amino acid sequence of SEQ ID NO: 1.

For example, a base sequence corresponding to the amino acid sequence of SEQ ID NO: 1 may be obtained by means of known software or web service for converting amino acid sequences to base sequences, and DNA having the obtained base sequence may be chemically synthesized using a hitherto known DNA synthesizer. As of the filing of the present application, some commercial services were known to which the steps of obtaining a base sequence from a hitherto known amino acid sequence and synthesizing DNA having the base sequence can be entrusted. In Example described later, such a commercial service was used to obtain the DNA encoding the amidohydrolase used in the present disclosure.

In production of the amidohydrolase used in the present disclosure, for example, a hitherto known autonomously-replicating vector can be used. More specifically, the DNA encoding the amidohydrolase used in the present disclosure and the autonomously-replicating vector may be used to construct replicable recombinant DNA, and the recombinant DNA may be introduced into host cells.

Typical examples of the autonomously-replicating vector used in the present disclosure include hitherto known plasmid vectors. Specific examples of the plasmid vectors include: pBR plasmids such as pBR322; pUC plasmids such as pUC18, pUC19, pUC118, and pUC119; pBS plasmids such as pBlueScript II, pBluescript II SK(+/-), pBluescript II KS (+/-), pBluescript II XR, and pBluescript II RI; pET plasmids such as pET-3a to 3d, pET-11a to 11d, pET-14b, pET-15b, and pET-21a to 21d; pGEX plasmids such as pGEX-1, pGEX-2T, and pGEX-3X; pTZ plasmids such as pTZ4, pTZ5, pTZ12, pTZ-18R, and pTZ-19R; pSU plasmids such as pSU0, pSU7, pSU22, and pSU23; *Bacillus* plasmids such as pUB110, pC194, pHY plasmids, pNU plasmids, pNY326, and pNC plasmids; and shuttle vector plasmids such as pHV14, TRp7, YEp plasmids, and pBS7.

Any of these plasmids can be selected depending on various factors such as the type of the host cells and the type of the expression system. The autonomously-replicating vector may be a phage vector.

The method for inserting the DNA encoding the amidohydrolase used in the present disclosure into the autonomously-replicating vector is not limited to using a particular technique, and any hitherto known method can be suitably used. For example, a commonly used method is, but not limited to, a method in which the DNA (or gene) encoding the amidohydrolase and the vector are digested (fragmented) with a hitherto known type-II restriction enzyme, then the resulting DNA fragment and vector fragment are annealed if necessary, and the fragments are ligated using a DNA ligase or the like.

The replicable recombinant DNA constructed as above may include another type of DNA in addition to the DNA encoding the amidohydrolase used in the present disclosure and the autonomously-replicating vector. For example, the replicable recombinant DNA may include DNA encoding a regulatory sequence that is not contained in the autonomously-replicating vector or may include DNA (or gene) encoding another protein or peptide. In this case, the amidohydrolase used in the present disclosure may be incorporated into the replicable recombinant DNA such that the amidohydrolase forms a chimeric protein together with the other protein or peptide.

The host cells used in production of the amidohydrolase used in the present disclosure, namely the host cells into which is introduced the recombinant DNA including the DNA encoding the amidohydrolase used in the present disclosure, are not limited to a particular type. Examples of commonly used cells include cells of microorganisms such as *Escherichia coli, Bacillus subtilis, Actinomycetes,* and yeasts. The host cells are not limited to these microbial cells and may be plant cells or animal cells. In Example described later, *Escherichia coli* cells were used as the host cells.

The method for introducing the recombinant DNA into the host cells, namely the transformation method, is not limited to using a particular technique, and any hitherto known method can be suitably used depending on the type of the host cells, the type of the autonomously-replicating vector, or any other factor. Typical examples of the transformation method used when the host cells are cells of a bacterium such as *Escherichia coli* include electroporation and a method in which the cells are converted into competent cells by means of calcium chloride. When the host cells are yeast cells, a method may be used in which the yeast cells are converted into spheroplasts by partially removing the cell walls of the yeast cells, or a lithium acetate method may be used. When the host cells are fungal cells, plant cells, or animal cells, a particle gun method or transfection method may be used.

A typical example of the method for producing the amidohydrolase used in the present disclosure is one in which the DNA encoding the amidohydrolase is introduced into the host cells by any of various methods as described above to prepare transformed cells and in which the transformed cells are cultured. In production of the amidohydrolase used in the present disclosure, the culture of the host cells is not limited to a particular scale. For example, in the case of using a liquid medium (culture fluid), small-scale culture using a test tube or flask may be carried out, large-scale culture using ajar fermenter may be carried out, or an industrial large-scale culture using a tank may be carried out.

The method for extracting the amidohydrolase used in the present disclosure from the cultured cells is not limited to using a particular technique, and any hitherto known method can be used. When the amidohydrolase expressed is accumulated in the cells, the cultured cells may be collected and disrupted by a hitherto known method to obtain a crude enzyme solution, and the crude enzyme solution may be purified or concentrated by a hitherto known method to extract the amidohydrolase. If there is no need for purification or concentration, the crude enzyme solution may be used as the amidohydrolase according to the present disclosure. When the amidohydrolase expressed is significantly secreted out of the cells, the amidohydrolase may be extracted from the whole culture system including the cultured cells and the culture fluid.

The method for amidohydrolase purification in production of the amidohydrolase used in the present disclosure is not limited to using a particular technique, and any hitherto known purification method can be suitably used. Examples of particularly suitable methods for protein purification include a method described in the following literatures: Nishiya, Y, Yamamoto, M., Takemoto, J., Kano, S., and Nakano, S., "Monomeric sarcosine oxidase exhibiting high substrate affinity and thermostability" Int. J. Anal. Bio-Sci., 4, 55-62 (2016) (Reference Literature 2); Hiruta, M. and Nishiya, Y, "Creation of an L-mandelate oxidase via structure-guided design of engineered lactate oxidase" Int. J. Anal. Bio-Sci., 6, 25-29 (2018) (Reference Literature 3); and Shimozawa, Y, Yoshida, S., Ikeda, K., Kato, Y., Toyama, F., and Nishiya, Y, "Easy preparation of a stable membrane-bound lactate dehydrogenase for application on lactate biosensor" Int. J. Anal. Bio-Sci., 8, 65-70 (2020) (Reference Literature 4). The contents of Reference Literatures 2 to 4 are incorporated herein by reference.

The method employed to produce the amidohydrolase used in the present disclosure may be any of various methods described in known literatures. A commercial service to which enzyme production can be entrusted and which was known as of the filing of the present application may also be used to produce the amidohydrolase. As previously stated, the production of the DNA encoding the amidohydrolase used in the present disclosure can be entrusted to a commercial service. A commercial service is also known to which the steps of constructing the recombinant DNA including the DNA encoding the amidohydrolase and constructing the overexpression system can be entrusted or the step of producing a protein by fermentation using microbial cells as host cells can be entrusted. The teachings of the present disclosure can be implemented using such a commercial service.

### [Typical Example of Glycine Measurement Method]

The following will describe a typical glycine measurement method used in the material detection method according to the present disclosure.

Glycine is an amino acid that has the simplest structure and that does not take stereoisomeric forms such as D- and L-forms. Glycine is one of the amino acids which constitute proteins and is known as a material for biosynthesis of various biological substances. It is also known that glycine may be formed as a by-product in various chemical reactions.

In some chemical reactions, it is difficult to directly detect the main products of the chemical reactions. In the case of a chemical reaction where glycine is formed as a by-product, whether a starting material is present in a sample or whether a main product is formed as a result of the chemical reaction of the starting material can be indirectly determined by detecting the glycine formed.

As previously mentioned, the present inventors have proposed a material detection method using glycine oxidase, and this method is disclosed in Patent Literature 1. In the present disclosure, the glycine measurement method used in the material detection method of Patent Literature 1 can be suitably used for detection of hippuric acid or the like. The contents of Patent Literature 1 (Japanese Laid-Open Patent Application Publication No. 2019-187285) are incorporated herein by reference.

A description will be given of an example where the glycine measurement method disclosed in Patent Literature 1 is used in the material detection method according to the present disclosure. First, as shown in FIG. 1 or the top of FIG. 2, a sample which may contain hippuric acid or the like is treated with the above-described aminoacylase (amidohydrolase having the amino acid sequence of SEQ ID NO: 1 or a homologous sequence). For the sake of convenience, the sample that has yet to be treated with the aminoacylase will be referred to as "primary sample". If the primary sample contains hippuric acid or the like, benzoic acid or the like and glycine are formed. In the present disclosure, the glycine formed is measured to detect (measure or quantify) hippuric acid or the like which is the starting material.

In the present embodiment, the glycine measurement method used is one in which, as shown in the middle and bottom of FIG. 2, hydrogen peroxide formed under the catalytic action of glycine oxidase is measured. For the sake of convenience, the sample resulting from the treatment of the primary sample with the aminoacylase will be referred to as "secondary sample".

The secondary sample resulting from the treatment with the aminoacylase, namely the sample which contains (or may contain) glycine, is treated with glycine oxidase. For the sake of convenience, the sample resulting from the treatment of the secondary sample with glycine oxidase will be referred to as "tertiary sample". If the secondary sample contains glycine, the glycine is oxidized and thus, as shown in the middle of the FIG. 2, hydrogen peroxide is directly formed along with glyoxylic acid and ammonia. That is, the tertiary sample contains directly-formed hydrogen peroxide.

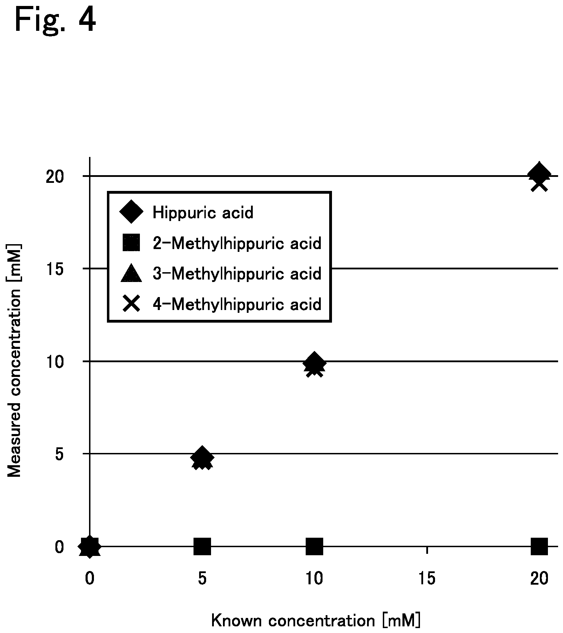

Subsequently, as shown in the bottom of FIG. 2, 4-aminoantipyrine (4-AA), a Trinder reagent, and a peroxidase are added and mixed with the tertiary sample resulting from the treatment with glycine oxidase. If the tertiary sample contains hydrogen peroxide (in other words, if the secondary sample that has yet to be treated with glycine oxidase contains glycine), the hydrogen peroxide reacts with the 4-AA and the Trinder reagent under the catalytic action of the peroxidase, giving a quinone pigment. For the sake of convenience, the sample resulting from the treatment of the tertiary sample with the peroxidase will be referred to as "quaternary sample".

The degree of coloring attributed to the quinone pigment can be measured, for example, based on the absorbance at 500 to 550 nm. Thus, the amount of formed hydrogen peroxide in the tertiary sample can be determined by measuring the absorbance of the quaternary sample. If the hydrogen peroxide in the tertiary sample can be quantified, this means that glycine contained in the secondary sample, namely the sample that has yet to be treated with glycine oxidase, can be measured (quantified or detected). If glycine in the secondary sample can be measured, this means that hippuric acid or the like contained in the primary sample, namely the initial sample that has yet to be treated with the aminoacylase, can be measured (quantified or detected).

Glycine oxidase (EC 1.4.3.19) used in the glycine measurement method as illustrated in FIG. 2 catalyzes deamination achieved by glycine oxidation. As shown by the formula (1) below, glycine oxidation leads to release of ammonia from glycine and formation of glyoxylic acid and hydrogen peroxide. Thus, the occurrence of glycine oxidation can be identified by detecting hydrogen peroxide. As is clear from the formula (1), oxidation of 1 mole of glycine leads to formation of 1 mole of hydrogen peroxide. Thus, the amount of glycine oxidized (the amount of glyoxylic acid formed) can be determined by determining the amount of hydrogen peroxide formed.

Glycine + H₂O + O₂ → Glyoxylic acid + NH₃ + H₂O₂ ••• (1)

The method for detecting hydrogen peroxide formed by the direct reaction of the formula (1) (hydrogen peroxide measurement method) is not limited to using a particular technique. In general, a method can be suitably used in which hydrogen peroxide, aminoantipyrine, and a Trinder reagent are reacted under the catalytic action of a peroxidase as shown in FIG. 2 and in which the degree of coloring attributed to the resulting quinone pigment is measured. This reaction is called Trinder reaction.

In the Trinder reaction, hydrogen peroxide in the sample acts as an oxidant, and the Trinder reagent acts as a hydrogen donor. 4-Aminoantipyrine (4-AA) is reacted with the hydrogen peroxide and the Trinder reagent through a catalytic reaction catalyzed by an oxidase. As a result of oxidative condensation, a quinone pigment is formed in the sample and thus the sample is colored. By measuring the degree of coloring using a device such as an absorbance detector, the amount of hydrogen peroxide in the sample and therefore the amount of glycine present before the enzymatic reaction can be determined.

The Trinder reagent is not limited to a particular type, and any hitherto known compound can be suitably used. Specific examples of the Trinder reagent include: aniline derivatives such as *N*-ethyl-*N*-(2-hydroxy-3-sulfopropyl)-3-methoxyaniline (TOOS), N-ethyl-N-sulfopropyl-3-methoxyaniline (ADPS), *N*-ethyl-*N*-sulfopropylaniline (ALPS), N-ethyl-N-sulfopropyl-3-methylaniline (TOPS), N-ethyl-N-(2-hydroxy-3-sulfopropyl)-3-methoxyaniline (ADOS), N-ethyl-N-(2-hydroxy-3-sulfopropyl)-3,5-dimethoxyaniline (DAOS), N-(2-hydroxy-3-sulfopropyl)-3,5-dimethoxyaniline (HDAOS), and *N*-ethyl-*N*-(2-hydroxy-3-sulfopropyl)-3,5-dimethylaniline (MAOS); phenol derivatives such as phenol, 4-chlorophenol, 2,4-dichlorophenol, 2,6-dichlorophenol, 3,5-dichlorophenol, 2,4-dibromophenol, 2,4,6-trichlorophenol, 2,4,6-tribromophenol, 3,5-dichloro-2-hydroxybenzenesulfonic acid, and 3-hydroxy-2,4,6-triiodobenzoic acid; and toluidine derivatives. TOOS is particularly suitable for use in the present disclosure.

The peroxidase used to form a quinone pigment from hydrogen peroxide is not limited to a particular type, and any hitherto known peroxidase can be suitably used. The quinone pigment formed is not limited to a particular quinone as the structure of the quinone pigment varies depending on the type of the compound used as the Trinder reagent. The quinone pigment measurement method is not limited to the absorbance measurement under the above-mentioned conditions as any other known technique can be used.

The glycine oxidase used in the glycine measurement method illustrated in the present embodiment is not limited to a particular type, and any hitherto known glycine oxidase can be suitably used. Specific examples of the glycine oxidase include that derived from *Bacillus cereus, Bacillus subtilis,* or *Geobacillus kaustophilus.*

Alternatively, in the glycine measurement method described above, the glycine oxidase-catalyzed enzymatic reaction shown in the middle of FIG. 2 may be replaced by another enzymatic reaction. The other enzymatic reaction is not limited to a particular reaction and may be any enzymatic reaction that forms hydrogen peroxide. Specific examples of the other enzymatic reaction include an enzymatic reaction using S-adenosyl-L-methionine, glycine-N-methyltransferase, and sarcosine oxidase, and such a reaction is described in Japanese Patent No. 5978658 (Reference Literature 5). The contents of Reference Literature 5 are incorporated herein by reference.

Generally, in the case of a conventional detection method, a glycine-sarcosine cycling reaction is used to amplify the measurement sensitivity for glycine, and this imposes the need to quantify glycine by a rate method.

Methods for detecting (measuring) materials by using enzymatic reactions can be classified into two types, a rate method and an endpoint method, which differ in analytical technique.

The rate method is a method in which a material is quantitatively detected by subjecting a substrate to a chemical reaction induced by the catalytic action of an enzyme and measuring the rate of progress of the reaction. In this method, for example, the rate of progress of the reaction catalyzed by the enzyme is measured in terms of the change in absorbance, turbidity, or any other property.

The endpoint method is a method in which a material is quantitatively detected by subjecting a substrate to a chemical reaction induced by the catalytic action of an enzyme, allowing the reaction of the substrate to proceed well (until the reaction reaches the endpoint), and then measuring the total amount of change after the reaction. In this method, for example, the reaction is allowed to proceed until the amount of the starting material which is the substrate substantially stops decreasing or until the amount of the product formed from the starting material substantially stops increasing, and after that the amount of the formed product is measured in terms of absorbance, turbidity, or any other property. In the endpoint method, the product formation by the chemical reaction gradually approaches a plateau. Thus, the endpoint method has the advantage of being more easily applicable to automatic measurement using a machine than the rate method.

In the present disclosure, as shown in FIG. 1 and the top of FIG. 2, a molar amount of glycine is formed as a by-product from the same molar amount of hippuric acid or the like. As shown in the middle of FIG. 2, the same molar amount of hydrogen peroxide is formed from the glycine. As shown in the bottom of FIG. 2, one molecule of quinone pigment is formed per two molecules of the hydrogen peroxide serving as an oxidant. Thus, the quinone pigment is formed in a molar amount equal to half the molar amount of hippuric acid or the like, and the endpoint method, rather than the rate method, can be suitably used. As previously stated, the endpoint method is easily applicable to measurement using a machine. Thus, with the use of the material detection method according to the present disclosure, detection (quantification) of hippuric acid or the like can be automatically carried out.

### [Utilization of Material Detection Method According to Present Disclosure]

As described above, the material detection method according to the present disclosure is a method for detecting a starting material by using a hitherto known technique to measure glycine formed as a by-product along with a main product through a chemical reaction involving the starting material and catalyzed by a hydrolase. The starting material includes hippuric acid and/or methylhippuric acid and the hydrolase is an amidohydrolase (aminoacylase) having the amino acid sequence of SEQ ID NO: 1 or a homologous sequence.

In the material detection method as defined above, the amidohydrolase having the amino acid sequence of SEQ ID NO: 1 or a homologous sequence is used as the enzyme that hydrolyzes hippuric acid or methylhippuric acid, and for this reason all of the three isomers of methylhippuric acid can be well hydrolyzed as well as hippuric acid. Thus, when a sample as the object of detection is enzymatically treated with the amidohydrolase used in the present disclosure, the treated sample contains glycine in an amount corresponding to the amount of hippuric acid and methylhippuric acid contained in the sample before the treatment. The total amount of hippuric acid and methylhippuric acid initially contained in the sample can be determined by measuring hydrogen peroxide.

If the amount of hippuric acid contained in the sample can be obtained, the amount of methylhippuric acid can be obtained by subtracting the amount of hippuric acid from the determined total amount of hippuric acid and methylhippuric acid. The method for determining the amount of hippuric acid which is to be subtracted from the total amount of hippuric acid and methylhippuric acid is not limited to using a particular technique, and any hitherto known technique can be suitably used. A typical example is a method in which the respective concentrations of hippuric acid and methylhippuric acid in a biological sample are measured, and this method is described in Japanese Patent No. 5751109 (Reference Literature 6). The contents of Reference Literature 6 are incorporated herein by reference.

As shown in Example described later, the use of the amidohydrolase according to the present disclosure yields the result that known and measured concentrations in a sample agree well with each other not only for hippuric acid but also for all of 2-methylhippuric acid, 3-methylhippuric acid, and 4-methylhippuric acid. In contrast, with the use of a hitherto known hydrolase such as aminoacylase PH1043 or hippurate hydrolase, known and measured concentrations are far different from each other for 2-methylhippuric acid although known and measured concentrations agree well with each other for hippuric acid, 3-methylhippuric acid, and 4-methylhippuric acid.

Reference Literature 1, which reports the amidohydrolase used in the present disclosure, fails to teach that the amidohydrolase has activity for hydrolysis of hippuric acid and methylhippuric acid. It remains unclear why the amidohydrolase used in the present disclosure, unlike aminoacylase PH1043 which is hitherto known, has enzymatic activity for 2-methylhippuric acid despite the fact that the amino acid sequence of the amidohydrolase is homologous to that of aminoacylase PH1043.

In the material detection method according to the present disclosure, the method for measuring glycine obtained by hydrolysis of hippuric acid or methylhippuric acid is not limited to using a particular technique. As previously stated, a typical method is one in which hydrogen peroxide formed under the catalytic action of glycine oxidase (or the catalytic action of another enzyme) is measured. With this measurement method, as previously stated, the endpoint method suitable for measurement using a machine can be employed. Thus, the use of the material detection method according to the present disclosure makes it possible to automatically carry out detection (quantification) of hippuric acid or the like.

The present disclosure covers not only the above-described material detection method itself but also various applications exploiting the material detection method. For example, the present disclosure covers a kit (material detection kit) for use in the above-described material detection method.

The material detection kit according to the present disclosure includes at least the amidohydrolase having the amino acid sequence of SEQ ID NO: 1 or a homologous sequence as the enzyme that hydrolyzes hippuric acid and methylhippuric acid which are starting materials. When included in the kit, the amidohydrolase may be prepared as an enzyme solution by a hitherto known technique. The enzyme solution is not limited to having a particular composition and may be composed of a hitherto known solvent and the amidohydrolase contained in the solvent at a concentration appropriate for the situation in which the enzyme solution is intended to be used.

The material detection kit according to the present disclosure may include, in addition to the amidohydrolase, other reagents or test instruments. In the material detection method according to the present disclosure, as previously stated, a glycine measurement method can be suitably used in which hydrogen peroxide is formed under the catalytic action of glycine oxidase (or the catalytic action of another enzyme) from glycine formed as a by-product under the catalytic action of the amidohydrolase and in which the hydrogen peroxide formed is detected. Thus, the material detection kit according to the present disclosure preferably includes glycine oxidase (or another enzyme) and preferably includes a reagent for detection of hydrogen peroxide.

Like the amidohydrolase, glycine oxidase (or another enzyme) may be prepared as an enzyme solution by a hitherto known technique. Examples of the reagent for detection of hydrogen peroxide include 4-aminoantipyrine, the Trinder reagent, and the peroxidase which are mentioned above. 4-Aminoantipyrine and the Trinder reagent may be prepared in the form of solutions by a hitherto known technique, and the peroxidase may also be prepared as an enzyme solution by a hitherto known technique.

As previously stated, the material detection method according to the present disclosure is applicable to an automatic measurement system. Thus, the material detection kit according to the present disclosure may include various test instruments that can be used in the automatic measurement system, and the amidohydrolase or the other reagents may be prepared in a hitherto known form that can be used in the automatic measurement system.

The other reagents included in the material detection kit according to the present disclosure may include a diluent for specimens, a diluent for enzymatic reactions, and buffers. Ordinarily, each of these reagents is individually put in a suitable sealed receptacle, which is included in the material detection kit. Examples of the test instruments that may be included in the material detection kit according to the present disclosure include, but are not limited to: hitherto known disposable instruments used for sampling from specimens; and microtubes for mixing the reagents. The material detection kit according to the present disclosure may further include an instruction manual that describes protocols required to use the material detection kit. The instruction manual may be in the form of a printed matter or may be recorded as data in a hitherto known recording medium.

Specific examples of the type of the material detection kit according to the present disclosure include a kit for obtaining a specimen from a worker who may handle toluene or xylene and detecting hippuric acid contained as a metabolite of toluene in the specimen or methylhippuric acid contained as a metabolite of xylene in the specimen or determining the total amount of hippuric acid and methylhippuric acid contained as metabolites in the specimen. Alternatively, the material detection kit according to the present disclosure may be a research or investigation kit for detecting or quantifying hippuric acid or methylhippuric acid that is not a metabolite.

The amidohydrolase having the amino acid sequence of SEQ ID NO: 1 or a homologous sequence, which is used in the material detection method or material detection kit according to the present disclosure, can be detected by using hippuric acid and methylhippuric acid as detection reagents. As previously stated, the amidohydrolase used in the present disclosure, like conventional enzymes, hydrolyzes hippuric acid, 3-methylhippuric acid, and 4-methylhippuric acid to form benzoic acid or methylbenzoic acid and glycine as a by-product. Furthermore, the amidohydrolase is capable of hydrolyzing 2-methylhippuric acid which cannot be well hydrolyzed by any conventional enzymes.

With the use of the material detection method according to the present disclosure, in which the aminoacylase (amidohydrolase) is used, the total amount of hippuric acid and methylhippuric acid in a sample can be determined by quantifying glycine. Alternatively, for example, the total amount of hippuric acid and methylhippuric acid in a sample can be determined also by quantifying benzoic acid or methylbenzoic acid. Thus, hippuric acid and methylhippuric acid can be used as detection reagents to detect the aminoacylase (amidohydrolase) used in the present disclosure.

### Examples

The present invention will be described in more detail based on Example and Comparative Examples. The present invention is not limited to Example presented below. A person skilled in the art can make various changes, modifications, or alterations without departing from the scope of the present invention.

### (Example)

A first reagent R1 was prepared which contained 30 units/mL of amidohydrolase 4ewt, 7.5 units/mL of peroxidase, 4.5 mM of N-ethyl-N-(2-hydroxy-3-sulfopropyl)-3-methoxyaniline (TOOS), and 50 mM of Tris buffer (pH 8.5), and a second reagent R2 was prepared which contained 5 units/mL of glycine oxidase, 1.2 mM of 4-aminoantipyrine, and 100 mM of Tris buffer (pH 8.5).

Specimen Groups 1 to 4 and Specimen 0 were prepared as measurement samples, i.e., examination specimens. As shown in Table 1, Specimen Group 1 is a group of specimens containing hippuric acid, Specimen Group 2 is a group of specimens containing 2-methylhippuric acid, Specimen Group 3 is a group of specimens containing 3-methylhippuric acid, Specimen Group 4 is a group of specimens containing 4-methylhippuric acid, and Specimen 0 is a negative control that contains neither hippuric acid nor methylhippuric acid. As shown in Table 1, each of Specimen Groups 1 to 4 consists of three specimens each of which is composed of a saline solution and 5 mM, 10 mM, or 20 mM of hippuric acid or methylhippuric acid contained in the saline solution.

**[Table 1]**

| Examination specimens | | Type | Concentration |
|---|---|---|---|
| Specimen Group 1 | Specimen 1-1 | Hippuric acid | 5 mM |
| | Specimen 1-2 | Hippuric acid | 10 mM |
| | Specimen 1-3 | Hippuric acid | 20 mM |
| Specimen Group 2 | Specimen 2-1 | 2-Methylhippuric acid | 5 mM |
| | Specimen 2-2 | 2-Methylhippuric acid | 10 mM |
| | Specimen 2-3 | 2-Methylhippuric acid | 20 mM |
| Specimen Group 3 | Specimen 3-1 | 3-Methylhippuric acid | 5 mM |
| | Specimen 3-2 | 3-Methylhippuric acid | 10 mM |
| | Specimen 3-3 | 3-Methylhippuric acid | 20 mM |
| Specimen Group 4 | Specimen 4-1 | 4-Methylhippuric acid | 5 mM |
| | Specimen 4-2 | 4-Methylhippuric acid | 10 mM |
| | Specimen 4-3 | 4-Methylhippuric acid | 20 mM |
| Specimen 0 | | Saline solution | - |

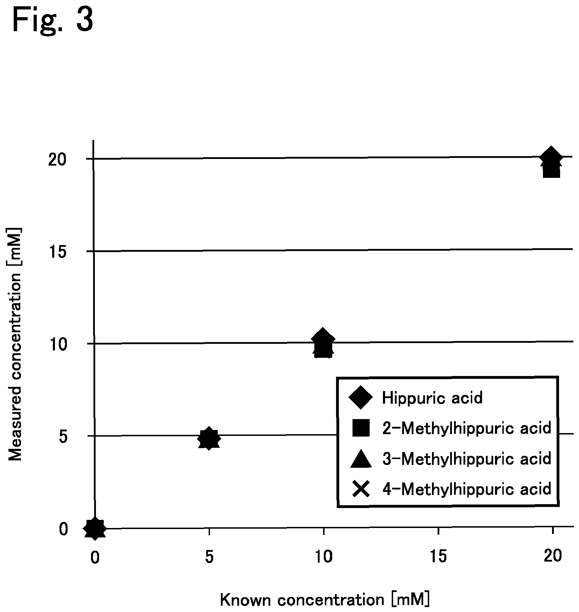

Each of the examination specimens was diluted to five times by means of an automatic analyzer to prepare a diluted specimen solution. A volume of 60 µL of the first reagent R1 was added and mixed with 2 µL of the diluted specimen solution, and the reaction was allowed to proceed well at 37°C. After that, 20 µL of the second reagent R2 was further added and mixed with the reaction mixture, and the reaction was allowed to proceed well at 37°C. The absorbance of the resulting specimen solution was measured using an automatic analyzer, manufactured by JEOL Ltd. under the trade name JCA-BM 6050, at a primary wavelength of 545 nm and a secondary wavelength of 805 nm. The concentration of hippuric acid or methylhippuric acid in each examination specimen was calculated from the measured absorbance and plotted on a graph. The results are shown in FIG. 3.

In FIG. 3, the abscissa represents known concentrations (unit: mM) of hippuric acid or methylhippuric acid in the examination specimens and the ordinate represents measured concentrations (unit: mM) obtained by the automatic analyzer. In FIG. 3, the diamond symbols represent the specimens of Specimen Group 1, namely the examination specimens containing hippuric acid, the square symbols represent the specimens of Specimen Group 2, namely the examination specimens containing 2-methylhippuric acid, the triangle symbols represent the specimens of Specimen Group 3, namely the examination specimens containing 3-methylhippuric acid, and the × symbols represent the specimens of Specimen Group 4, namely the examination specimens containing 4-methylhippuric acid. The examination specimen for which the known concentration is 0 mM is Specimen 0, which is represented by using all of the symbols representing Specimen Groups 1 to 4 since, as previously stated, Specimen 0 was used as a negative control in measurement for the Specimen Groups 1 to 4.

The amidohydrolase 4ewt used in Example was produced using a gene chemically synthesized by converting the amino acid sequence of SEQ ID NO: 1 to a base sequence. Specifically, 4ewt gene was prepared through GenScript Japan (URL:https://www.genscript.jp/gene_synthesis.html) which is a commercial service for artificial gene synthesis and, as previously described, the 4ewt gene was incorporated into a hitherto known vector to prepare recombinant DNA, the recombinant DNA was introduced into *Escherichia coli* cells, 4ewt protein was overexpressed in the cells, and the overexpressed protein was purified by the hitherto known method described in Reference Literatures 2 to 4 mentioned above. This is how the amidohydrolase 4ewt used in Example was produced.

### (Comparative Example 1)

The absorbance of each specimen was measured in the same manner as in Example, except for using hitherto known aminoacylase PH 1043 instead of the amidohydrolase used in Example. The concentration of hippuric acid or methylhippuric acid in each examination specimen was calculated from the measured absorbance and plotted on a graph. The results are shown in FIG. 4. The abscissa, ordinate, and symbols in FIG. 4 are the same as those in FIG. 3.

The aminoacylase PH1043 used in Comparative Example 1 is one produced by Nipro Corporation. The aminoacylase PH1043 will not be described in detail as it is a hitherto known enzyme described, for example, in the following literatures: Imai Satsuki, Nagoshi Kentaro, Shinki Shota, Baba Toshiaki, Nishiya Yoshiaki, "Functional Modification of Pyrococcus horikoshii-Derived Hippurate Hydrolase", The Society for Biotechnology, Japan (2018) (Reference Literature 7); Imai Satsuki, Nagoshi Kentaro, Shinki Shota, Baba Toshiaki, Nishiya Yoshiaki, "Development of Method and Enzyme for Enzymatic Assay of Hippuric Acid", The Society of Analytical Bio-Science (2019) (Reference Literature 8); Imai Satsuki, Nagoshi Kentaro, Shinki Shota, Baba Toshiaki, Nishiya Yoshiaki, "Functional Improvement of Hippurate Hydrolase", Japan Society for Bioscience, Biotechnology and Agrochemistry (2019) (Reference Literature 9); Imai Satsuki, Nagoshi Kentaro, Shinki Shota, Baba Toshiaki, Nishiya Yoshiaki, "Functional Improvement of Hippurate Hydrolase toward Practical Use", The Molecular Biology Society of Japan (2019) (Reference Literature 10); Imai Satsuki, Nagoshi Kentaro, Shinki Shota, Baba Toshiaki, Nishiya Yoshiaki, "Development of Enzymatic Assay Method for Determining Total Exposure to Toluene and Xylene (1)", The Society of Analytical Bio-Science (2020) (Reference Literature 11); and Suginaka Muneyuki, Torii Gento, Imai Satsuki, Baba Toshiaki, Nishiya Yoshiaki, "Development of Enzymatic Assay Method for Determining Total Exposure to Toluene and Xylene (2)", The Molecular Biology Society of Japan (2020) (Reference Literature 12). The contents of Reference Literatures 7 to 12 are incorporated herein by reference.

### (Comparative Example 2)

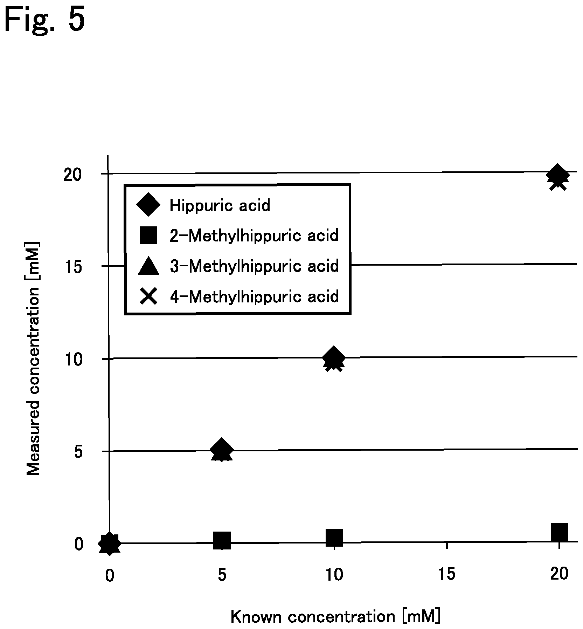

The absorbance of each specimen was measured in the same manner as in Example, except for using a hitherto known hippurate hydrolase (EC 3.5.1.32, manufactured by Nipro Corporation) instead of the amidohydrolase used in Example. The concentration of hippuric acid or methylhippuric acid in each examination specimen was calculated from the measured absorbance and plotted on a graph. The results are shown in FIG. 5. The abscissa, ordinate, and symbols in FIG. 5 are the same as those in FIG. 3 or 4.

### (Comparison between Example and Comparative Examples)

As is clear from the results of Example, when the amidohydrolase having the amino acid sequence of SEQ ID NO: 1 was used, the measured concentrations in the specimens agreed well with the actual concentrations in the specimens for all of Specimen Groups 1 to 4.

In Comparative Example 1 where aminoacylase PH1043 was used and Comparative Example 2 where a hitherto known hippurate hydrolase was used, the measured concentrations in the specimens agreed well with the actual concentrations in the specimens for Specimen Groups 1, 3, and 4, namely for hippuric acid, 3-methylhippuric acid, and 4-methylhippuric acid. However, for Specimen Group 2, namely for 2-methylhippuric acid, the measured concentrations were not much different from the measured concentration obtained for the negative control (Specimen 0).

This reveals that the amidohydrolase used in the present disclosure exhibits well reactivity with all of hippuric acid and the three types of methylhippuric acid, whereas the conventional aminoacylase or hippurate hydrolase cannot exhibit sufficient reactivity with 2-methylhippuric acid. The material detection method according to the present disclosure has been demonstrated to be able to well determine the total amount of hippuric acid and methylhippuric acid. With the use of the material detection method according to the present disclosure, the amount of methylhippuric acid can be determined by subtracting the amount of hippuric acid from the total amount of hippuric acid and methylhippuric acid.

The present invention is not limited to the embodiment described above, and various modifications can be made without departing from the scope as defined by the appended claims. The technical scope of the present invention encompasses embodiments obtained by combining technical features disclosed in different embodiments or variants.

From the foregoing description, numerous modifications and other embodiments of the present invention are obvious to those skilled in the art. Accordingly, the foregoing description is to be construed as illustrative only, and is provided for the purpose of teaching those skilled in the art the best mode for carrying out the present invention. The structural and/or functional details may be substantially modified without departing from the scope of the present invention.

### Industrial Applicability

The present invention is suitable for use in a field where the presence of hippuric acid or methylhippuric acid as a starting material is detected by detecting glycine formed as a by-product through an enzymatic reaction catalyzed by an aminoacylase or in the field of material detection methods for determining the total amount of hippuric acid and methylhippuric acid.

## Claims

1. A material detection method comprising detecting at least a presence of a starting material by measuring glycine formed as a by-product along with a main product through a chemical reaction involving the starting material and catalyzed by a hydrolase, wherein
the starting material comprises hippuric acid and/or methylhippuric acid, and
the hydrolase is an aminoacylase having an amino acid sequence of SEQ ID NO: 1 or a protein having the amino acid sequence of SEQ ID NO: 1 in which one or more amino acids are deleted, replaced, or added and having activity for hydrolysis of the hippuric acid and the methylhippuric acid.

2. The material detection method according to claim 1, wherein the measuring of the glycine is accomplished by measuring hydrogen peroxide formed from the glycine.

3. The material detection method according to claim 2, wherein the hydrogen peroxide is formed by an enzymatic reaction catalyzed by glycine oxidase.

4. The material detection method according to claim 3, wherein the measuring of the hydrogen peroxide is accomplished by reacting the hydrogen peroxide, aminoantipyrine, and a Trinder reagent under catalytic action of a peroxidase to form a quinone pigment and by subsequently measuring a degree of coloring attributed to the quinone pigment.

5. The material detection method according to any one of claims 1 to 4, wherein the main product comprises benzoic acid or methylbenzoic acid.

6. The material detection method according to any one of claims 1 to 5, wherein a total amount of the hippuric acid and the methylhippuric acid contained in a sample that is an object of detection is determined by the measuring of the hydrogen peroxide.

7. The material detection method according to claim 6, wherein an amount of the methylhippuric acid is obtained by subtracting an amount of the hippuric acid from the determined total amount of the hippuric acid and the methylhippuric acid.

8. A material detection kit for use in the material detection method according to any one of claims 1 to 7, the material detection kit comprising an enzyme that hydrolyzes the starting material, wherein
the enzyme is an aminoacylase having an amino acid sequence of SEQ ID NO: 1 or a protein having the amino acid sequence of SEQ ID NO: 1 in which one or more amino acids are deleted, replaced, or added and having activity for hydrolysis of the hippuric acid and the methylhippuric acid.
